# FASCICULE DE BREVET EUROPEEN

(11) **EP 0 966 253 B1**
(45) Date de publication et mention de la délivrance du brevet: **22.10.2003**
(21) Numéro de dépôt: 98942731.5
(22) Date de dépôt: 11.08.1998
(51) Int. Cl.: A61K 7/13, D06P 1/00

(54) **COMPOSITION DE TEINTURE D'OXYDATION DES FIBRES KERATINIQUES**
ZUSAMMENSETZUNG FÜR DIE OXIDATIONSFÄRBUNG VON KERATINISCHEN FASERN
OXIDATION DYEING COMPOSITION FOR KERATIN FIBRES

(30) Priorité: 23.09.1997 FR 9711823
(43) Date de publication de la demande: 29.12.1999
(73) Titulaire: L'OREAL, 75008 Paris (FR)
(72) Inventeur: MAUBRU, Mireille, F-78400 Chatou (FR)
(74) Mandataire: Goulard, Sophie
(86) Numéro de dépôt international: FR9801796
(87) Numéro de publication internationale: WO99015140

(56) Documents cités:
- EP-A- 0 716 846
- EP-A- 0 795 313

## Description

L'invention a pour objet une composition pour la teinture d'oxydation des fibres kératiniques, et en particulier des fibres kératiniques humaines telles que les cheveux comprenant, dans un milieu approprié pour la teinture, au moins une base d'oxydation, de la choline oxydase, et au moins un donneur pour ladite choline oxydase, ainsi que le procédé de teinture mettant en oeuvre cette composition.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des bases hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

La coloration d'oxydation des fibres kératiniques est généralement réalisée en milieu alcalin, en présence de peroxyde d'hydrogène. Toutefois, l'utilisation des milieux alcalins en présence de peroxyde d'hydrogène présentent pour inconvénient d'entraîner une dégradation non négligeable des fibres, ainsi qu'une décoloration des fibres kératiniques qui n'est pas toujours souhaitable.

La coloration d'oxydation des fibres kératiniques peut également être réalisée à l'aide de systèmes oxydants différents du peroxyde d'hydrogène tels que des systèmes enzymatiques. Ainsi il a déjà été proposé de teindre les fibres kératiniques, notamment dans la demande de brevet EP-A-0 310 675, avec des compositions comprenant un précurseur de colorant d'oxydation de type benzénique, en association avec des enzymes telles que la pyranose-oxydase, la glucose-oxydase ou bien l'uricase, en présence d'un donneur pour lesdites enzymes. La demande de brevet EP-A-0 795 313 décrit une composition pour la teinture d'oxydation des fibres kératiniques comprenant une base d'oxydation, une coupleur choisi parmi des métaphénylènediamines particulières, une enzyme oxydase et un donneur pour cette enzyme et une enzyme peroxydase.

Dans la demande de betwet EP-A-0 716 846, il a été proposé, pour la coloration d'oxydation des fibres kératiniques, une composition comprenant au moins un colorant d'oxydation, de l'uricase et de l'acide urique.

Ces procédés de teinture, bien qu'étant mis en oeuvre dans des conditions n'entraînant pas une dégradation des fibres kératiniques comparable à celle engendrée par les teintures réalisées en présence de peroxyde d'hydrogène, ne sont pas entièrement satisfaisantes, notamment en ce qui concerne la puissance des colorations obtenues.

Or, la demanderesse vient maintenant de découvrir qu'il est possible d'obtenir de nouvelles teintures, capables de conduire à des colorations plus puissantes que celles de l'art antérieur mettant en oeuvre un système enzymatique, en associant au moins une base d'oxydation, au moins une enzyme de type choline oxydase, et au moins un donneur pour ladite enzyme.

Cette découverte est à la base de la présente invention.

L'invention a donc pour premier objet une composition prête à l'emploi, pour la teinture d'oxydation des fibres kératiniques et en particulier des fibres kératiniques humaines telles que les cheveux, caractérisée par le fait qu'elle comprend, dans un milieu approprié pour la teinture :
- au moins une base d'oxydation,
- de la choline oxydase,
- et au moins un donneur pour ladite choline oxydase.

La composition tinctoriale conforme à l'invention permet d'obtenir des colorations plus puissantes que celles obtenues avec les compositions de l'art antérieur utilisant un système oxydant enzymatique, tel que par exemple le système oxydant acide urique / uricase.
De plus, les colorations obtenues avec la composition tinctoriale conforme à l'invention sont peu sélectives et résistent bien aux différentes agressions que peuvent subir les cheveux, (lumière, intempéries, lavages, déformations permanentes, etc...).

L'invention a également pour objet un procédé de teinture d'oxydation des fibres kératiniques mettant en oeuvre cette composition tinctoriale prête à l'emploi.

La nature de la ou des bases d'oxydation utilisées dans la composition tinctoriale prête à l'emploi n'est pas critique. Elles peuvent notamment être choisies parmi les paraphénylènediamines, les bases doubles, les paraaminophénols, les ortho aminophénols et les bases d'oxydation hétérocycliques.

Parmi les paraphénylènediamines utilisables à titre de base d'oxydation dans les composition tinctoriales conformes à l'invention, on peut notamment citer les composés de formule (I) suivante et leurs sels d'addition avec un acide : 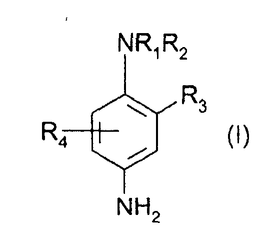 dans laquelle :
- R₁ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), alkyle en C₁-C₄ substitué par un groupement azoté, phényle ou 4'-aminophényle ;
- R₂ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄) ou alkyle en C₁-C₄ substitué par un groupement azoté ;
- R₃ représente un atome d'hydrogène, un atome d'halogène tel qu'un atome de chlore, de brome, d'iode ou de fluor, un radical alkyle en C₁-C₄, monohydroxyalkyle en C₁-C₄, hydroxyalcoxy en C₁-C₄, acétylaminoalcoxy en C₁-C₄, mésylaminoalcoxy en C₁-C₄ ou carbamoylaminoalcoxy en C₁-C₄,
- R₄ représente un atome d'hydrogène, d'halogène ou un radical alkyle en C₁-C₄.

Parmi les groupements azotés de la formule (I) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les paraphénylènediamines de formule (I) ci-dessus, on peut plus particulièrement citer la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-methoxyéthyl) paraphénylènediamine, et leurs sels d'addition avec un acide.

Parmi les paraphénylènediamines de formule (I) ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide.

Selon l'invention, on entend par bases doubles, les composés comportant au moins deux noyaux aromatiques sur lesquels sont portés des groupements amino et/ou hydroxyle.

Parmi les bases doubles utilisables à titre de bases d'oxydation dans les compositions tinctoriales conformes à l'invention, on peut notamment citer les composés répondant à la formule (II) suivante, et leurs sels d'addition avec un acide : 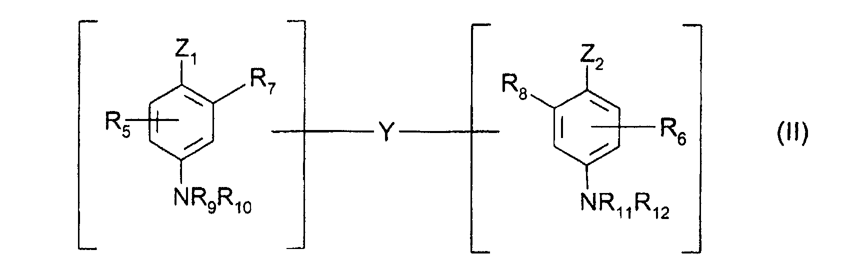 dans laquelle :
- Z₁ et Z₂, identiques ou différents, représentent un radical hydroxyle ou -NH₂ pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
- R₅ et R₆ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₇, R₈, R₉, R₁₀ R₁₁ et R₁₂, identiques ou différents, représentent un atome d'hydrogène, un bras de liaison Y ou un radical alkyle en C₁-C₄ ;
étant entendu que les composés de formule (II) ne comportent qu'un seul bras de liaison Y par molécule.

Parmi les groupements azotés de la formule (II) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les bases doubles de formules (II) ci-dessus, on peut plus particulièrement citer le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi ces bases doubles de formule (II), le N,N'-bis-(β-hydroxyéthyl) N,N'-bis-(4'-aminophényl) 1,3-diamino propanol, le 1,8-bis-(2,5-diaminophénoxy)-3,5-dioxaoctane ou l'un de leurs sels d'addition avec un acide sont particulièrement préférés.

Parmi les para-aminophénols utilisables à titre de bases d'oxydation dans les compositions tinctoriales conformes à l'invention, on peut notamment citer les composés répondant à la formule (III) suivante, et leurs sels d'addition avec un acide : 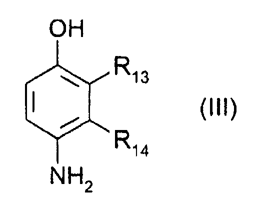 dans laquelle :
- R₁₃ représente un atome d'hydrogène ou d'halogène, un radical alkyle en C₁-C₄, monohydroxyalkyle en C₁-C₄, alcoxy(C₁-C₄)alkyle(C₁-C₄), aminoalkyle en C₁-C₄ ou hydroxyalkyl(C₁-C₄)aminoalkyle en C₁-C₄,
- R₁₄ représente un atome d'hydrogène ou d'halogène, un radical alkyle en C₁-C₄, monohydroxyalkyle en C₁-C₄, polyhydroxyalkyle en C₂-C₄, aminoalkyle en C₁-C_{4,} cyanoalkyle en C₁-C₄ ou alcoxy(C₁-C₄)alkyle(C₁-C₄),
étant entendu qu'au moins un des radicaux R₁₃ ou R₁₄ représente un atome d'hydrogène.

Parmi les para-aminophénols de formule (III) ci-dessus, on peut plus particulièrement citer le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les orthoaminophénols utilisables à titre de bases d'oxydation dans les compositions tinctoriales conformes à l'invention, on peut plus particulièrement citer le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, les dérivés pyrazolo-pyrimidiniques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevet allemand DE 2 359 399 ou la demande de brevet WO 96/15765, comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Parmi les dérivés pyrazolo-pyrimidiniques, on peut plus particulièrement citer les pyrazolo-[1,5-a]-pyrimidines de formule (IV) suivante, leurs sels d'addition avec un acide ou avec une base et leurs formes tautomères, lorsqu'il existe un équilibre tautomérique : 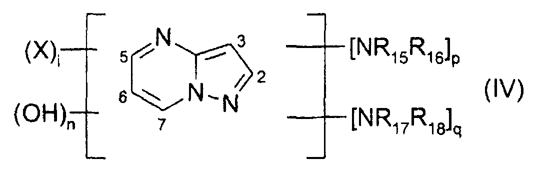 dans laquelle :
- R₁₅, R₁₆, R₁₇ et R₁₈, identiques ou différents désignent, un atome d'hydrogène, un radical alkyle en C₁-C₄, un radical aryle, un radical hydroxyalkyle en C₁-C₄, un radical polyhydroxyalkyle en C₂-C₄, un radical (C₁-C₄)alcoxy alkyle en C₁-C₄, un radical aminoalkyle en C₁-C₄ (l'amine pouvant être protégée par un radical acétyle, uréido ou sulfonyle), un radical (C₁-C₄)alkylamino alkyle en C₁-C₄, un radical di-[(C₁-C₄)alkyl] amino alkyle en C₁-C₄ (les radicaux dialkyles pouvant former un cycle carboné ou un hétérocycle à 5 ou 6 chaînons), un radical hydroxy(C₁-C₄)alkyl- ou di-[hydroxy(C₁-C₄) alkyl]-amino alkyle en C₁-C₄;
- les radicaux X désignent, identiques ou différents, un atome d'hydrogène, un radical alkyle en C₁-C₄, un radical aryle, un radical hydroxyalkyle en C₁-C₄, un radical polyhydroxyalkyle en C₂-C₄, un radical amino alkyle en C₁-C₄, un radical (C₁-C₄)alkyl amino alkyle en C₁-C₄, un radical di-[(C₁-C₄)alkyl] amino alkyle en C₁-C₄ (les dialkyles pouvant former un cycle carboné ou un hétérocycle à 5 ou 6 chaînons), un radical hydroxy(C₁-C4)alkyl ou di-[hydroxy(C₁-C₄ )alkyl]amino alkyle en C₁-C₄, un radical amino, un radical (C₁-C₄)alkyl- ou di-[(C₁-C₄)alkyl]-amino ; un atome d'halogène, un groupe acide carboxylique, un groupe acide sulfonique ;
- i vaut 0, 1, 2 ou 3 ;
- p vaut 0 ou 1 ;
- q vaut 0 ou 1 ;
- n vaut 0 ou 1 ;
sous réserve que :
- la somme p + q est différente de 0 ;
- lorsque p + q est égal à 2, alors n vaut 0 et les groupes NR₁₅R₁₆ et NR₁₇R₁₈ occupent les positions (2,3) ; (5,6) ; (6,7) ; (3,5) ou (3,7) ;
- lorsque p + q est égal à 1 alors n vaut 1 et le groupe NR₁₅R₁₆ (ou NR₁₇R₁₈) et le groupe OH occupent les positions (2,3) ; (5,6) ; (6,7) ; (3,5) ou (3,7) ;

Lorsque les pyrazolo-[1,5-a]-pyrimidines de formule (IV) ci-dessus sont telles qu'elles comportent un groupe hydroxyle sur l'une des positions 2, 5 ou 7 en α d'un atome d'azote, il existe un équilibre tautomérique représenté par exemple par le schéma suivant :

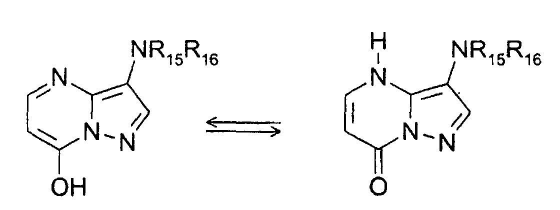

Parmi les pyrazolo-[1,5-a]-pyrimidines de formule (IV) ci-dessus on peut notamment citer :
- la pyrazolo-[1,5-a]-pyrimidine-3,7-diamine ;
- la 2,5-diméthyl pyrazolo-[1,5-a]-pyrimidine-3,7-diamine ;
- la pyrazolo-[1,5-a]-pyrimidine-3,5-diamine ;
- la 2,7-diméthyl pyrazolo-[1,5-a]-pyrimidine-3,5-diamine ;
- le 3-amino pyrazolo-[1,5-a]-pyrimidin-7-ol
- le 3-amino pyrazolo-[1,5-a]-pyrimidin-5-ol
- le 2-(3-amino pyrazolo-[1,5-a]-pyrimidin-7-ylamino)-éthanol
- le 2-(7-amino pyrazolo-[1,5-a]-pyrimidin-3-ylamino)-éthanol
- le 2-[(3-Amino-pyrazolo[1,5-a]pyrimidin-7-yl)-(2-hydroxy-éthyl)-amino]-éthanol
- le 2-[(7-Amino-pyrazolo[1,5-a]pyrimidin-3-yl)-(2-hydroxy-éthyl)-amino]-éthanol
- la 5,6-diméthyl pyrazolo-[1,5-a]-pyrimidine-3,7-diamine ;
- la 2,6-diméthyl pyrazolo-[1,5-a]-pyrimidine-3,7-diamine ;
- la 2, 5, N 7, N 7-tetraméthyl pyrazolo-[1,5-a]-pyrimidine-3,7-diamine ;
et leurs sels d'addition et leurs formes tautomères, lorsqu'il existe un équilibre tautomérique.

Les pyrazolo-[1,5-a]-pyrimidines de formule (IV) ci-dessus peuvent être préparées par cyclisation à partir d'un aminopyrazole selon les synthèses décrites dans les références suivantes :
- EP 628559 BEIERSDORF-LILLY
- R. Vishdu, H. Navedul, Indian J. Chem., 34b (6), 514, 1995.
- N.S. Ibrahim, K.U. Sadek, F.A. Abdel-AI, Arch. Pharm., 320, 240, 1987.
- R.H. Springer, M.B. Scholten, D.E. O'Brien, T. Novinson, J.P. Miller, R.K. Robins, J. Med. Chem., 25, 235, 1982.
- T. Novinson, R.K. Robins, T.R. Matthews, J. Med. Chem., 20, 296, 1977.
- US 3907799 ICN PHARMACEUTICALS

Les pyrazolo-[1,5-a]-pyrimidines de formule (IV) ci-dessus peuvent également être préparées par cyclisation à partir d'hydrazine selon les synthèses décrites dans les références suivantes :
- A. McKillop et R.J. Kobilecki, Heterocycles, 6(9), 1355, 1977.
- E. Alcade, J. De Mendoza, J.M. Marcia-Marquina, C. Aimera, J. Elguero, J. Heterocyclic Chem., 11(3), 423, 1974.
- K. Saito, I. Hori, M. Higarashi, H. Midorikawa, Bull. Chem. Soc. Japan, 47(2), 476, 1974.

La ou les bases d'oxydation conformes à l'invention représentent de préférence de 0,0005 à 12 % en poids environ du poids total de la composition tinctoriale prête à l'emploi, et encore plus préférentiellement de 0,005 à 6 % en poids environ de ce poids.

La choline oxydase utilisée dans la composition tinctoriale prête à l'emploi conforme à l'invention peut être d'origine animale, microbiologique (bactérienne, fongique ou virale) ou synthétique (obtenue par synthèse chimique ou biotechnologique).

A titre d'exemple de sources de choline oxydase, on peut notamment citer le foie de rat, les bactéries telles que Arthrobacter globiformis, Achromobacter cholinophagum ou Alcaligenes, et les champignons tels que Cylindrocarpon didynum.

La choline oxydase utilisée dans la composition tinctoriale prête à l'emploi conforme à l'invention représente de préférence de 0,01 à 20 % en poids environ du poids total de la composition tinctoriale prête à l'emploi, et encore plus préférentiellement de 0,1 à 5 % en poids environ de ce poids.

Selon l'invention, on entend par donneur, le ou les différents substrats nécessaires au fonctionnement de la choline oxydase.

Parmi les donneurs pour la choline oxydase, on peut mentionner la choline et ses sels d'addition avec un acide comme le chlorhydrate de choline, et la bétaïne aldéhyde.

Le ou les donneurs (ou substrats) utilisés conformément à l'invention représentent de préférence de 0,01 à 20 % en poids environ du poids total de la composition tinctoriale prête à l'emploi conforme à l'invention et encore plus préférentiellement de 0,1 à 5 % en environ de ce poids.

La composition tinctoriale prête à l'emploi conforme à l'invention peut également renfermer un ou plusieurs coupleurs et/ou un ou plusieurs colorants directs, notamment pour modifier les nuances ou les enrichir en reflets.

Parmi les coupleurs utilisables dans les compositions tinctoriales prêtes à l'emploi conformes à l'invention, on peut notamment citer les métaphénylènediamines, les méta-aminophénols, les métadiphénols et les coupleurs hétérocycliques tels que par exemple les dérivés indoliques, les dérivés indoliniques, les dérivés de benzimidazole, les dérivés de benzomorpholine, les dérivés de sésamol, les dérivés pyridiniques, pyrimidiniques et pyrazoliques, et leurs sels d'addition avec un acide.

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, et leurs sels d'addition avec un acide.

Lorsqu'ils sont présents ces coupleurs représentent de préférence de 0,0001 à 10 % en poids environ du poids total de la composition tinctoriale prête à l'emploi et encore plus préférentiellement de 0,005 à 5 % en poids environ de ce poids.

D'une manière générale, les sels d'addition avec un acide utilisables dans le cadre des compositions tinctoriales de l'invention (bases d'oxydation et coupleurs) sont notamment choisis parmi les chlorhydrates, les bromhydrates, les sulfates et les tartrates, les lactates et les acétates.

Le milieu approprié pour la teinture (ou support) de la composition tinctoriale prête à l'emploi conforme à l'invention est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition prête à l'emploi conforme à l'invention est choisi de telle manière que l'activité enzymatique de la choline oxydase soit suffisante. Il est généralement compris entre 5 et 11 environ, et de préférence entre 6,5 et 10 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines, le 2-méthyl 2-amino propanol ainsi que leurs dérivés, les hydroxydes de sodium ou de potassium et les composés de formule (V) suivante : 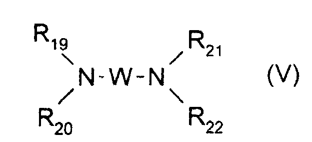 dans laquelle W est un reste propylène éventuellement substitué par un groupement hydroxyle ou un radical alkyle en C₁-C₄ ; R₁₉, R₂₀, R₂₁ et R₂₂, identiques ou différents, représentent un atome d'hydrogène, un radical alkyle en C₁-C₄ ou hydroxyalkyle en C₁-C₄.

La composition tinctoriale prête à l'emploi conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des enzymes différentes de la choline oxydase utilisée conformément à l'invention telles que par exemples des peroxydases, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Bien entendu, l'homme de l'art veillera à choisir ce ou ces éventuels composés complémentaires de manière telle que les propriétés avantageuses attachées intrinsèquement à la composition tinctoriale prête à l'emploi conforme à l'invention ne soient pas, ou substantiellement pas, altérées par la ou les adjonctions envisagées.

La composition tinctoriale prête à l'emploi conforme à l'invention peut se présenter sous des formes diverses, telles que sous forme de liquides, de crèmes, de gels, éventuellement pressurisés, ou sous toute autre forme appropriée pour réaliser une teinture des fibres kératiniques, et notamment des cheveux humains. Dans ce cas, le ou les colorants d'oxydation et la choline oxydase sont présents au sein de la même composition prête à l'emploi, et par conséquent ladite composition doit être exempte d'oxygène gazeux, de manière à éviter toute oxydation prématurée du ou des colorants d'oxydation.

L'invention a également pour objet un procédé de teinture des fibres kératiniques et en particulier des fibres kératiniques humaines telles que les cheveux mettant en oeuvre la composition tinctoriale prête à l'emploi telle que définie précédemment.

Selon ce procédé, on applique sur les fibres au moins une composition tinctoriale prête à l'emploi telle que définie précédemment, pendant un temps suffisant pour développer la coloration désirée, après quoi on rince, on lave éventuellement au shampooing, on rince à nouveau et on sèche.

Le temps nécessaire au développement de la coloration sur les fibres kératiniques est généralement compris entre 3 et 60 minutes et encore plus précisément 5 et 40 minutes.

Selon une autre forme de réalisation particulière de l'invention, le procédé comporte une étape préliminaire consistant à stocker sous forme séparée, d'une part, une composition (A) comprenant, dans un milieu approprié pour la teinture, au moins une base d'oxydation et, d'autre part, une composition (B) renfermant, dans un milieu approprié pour la teinture, de la choline oxydase en présence d'au moins un donneur pour ladite choline oxydase, puis à procéder à leur mélange au moment de l'emploi avant d'appliquer ce mélange sur les fibres kératiniques.

Un autre objet de l'invention est un dispositif à plusieurs compartiments ou "kit" de teinture ou tout autre système de conditionnement à plusieurs compartiments dont un premier compartiment renferme la composition (A) telle que définie ci-dessus et un second compartiment renferme la composition (B) telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

### EXEMPLES

### EXEMPLES 1 ET 2 COMPARATIFS

On a préparé les compositions tinctoriales prêtes à l'emploi suivantes (teneurs en grammes) :

| **COMPOSITION** | **1** | **2(*)** |
|---|---|---|
| Paraphénylènediamine (base d'oxydation) | 0,216 | 0,216 |
| Dichlorhydrate de 2,4-diaminophénoxy éthanol (coupleur) | 0,723 | 0,723 |
| Choline oxydase d'Alcaligenes à 18 Unités Internationales (U.I.) / mg, commercialisée par la société Sigma (enzyme conforme à l'invention) | 1,111 | - |
| Chlorhydrate de choline (donneur conforme à l'invention) | 0,166 | - |
| Uricase d'Arthrobacter globiformis à 20 Unités Internationales (U.I.) / mg, commercialisée par la société Sigma (enzyme hors invention) | - | 1,0 |
| Acide urique (donneur hors invention) | - | 0,2 |
| Ethanol | 10,0 | 10,0 |
| Monoéthanolamine q.s.p. | pH = 9,5 | pH = 9,5 |
| Eau déminéralisée q.s.p. | 100 g | 100 g |

| | | |
|---|---|---|
| (*) Exemple ne faisant pas partie de l'invention | | |

Il est important de noter que chacune des compositions tinctoriales prêtes à l'emploi décrites ci-dessus contient la même quantité d'enzyme, à savoir 20000 U.I..

Chacune des compositions tinctoriales prêtes à l'emploi décrites ci-dessus a été appliquée sur des mèches de cheveux gris permanentés à 90 % de blancs pendant 30 minutes. Les cheveux ont ensuite été rincés, lavés avec un shampooing standard, puis séchés.

La couleur des mèches a été ensuite évaluée avant et 24 heures après la coloration dans le système MUNSELL au moyen d'un colorimètre CM 2002 MINOLTA ® de façon à déterminer la puissance des colorations obtenues avec chacune des compositions décrites ci-dessus.

La différence entre la couleur de la mèche avant la teinture et la couleur de la mèche après la teinture a été calculée en appliquant la formule de NICKERSON :

**ΔE = 0,4 CoΔH + 6ΔV + 3 ΔC**

telle que décrite par exemple dans "Couleur, Industrie et Technique" ; pages 14-17 ; vol. n° 5 ; 1978.

Dans cette formule, ΔE représente la différence de couleur entre deux mèches, ΔH, ΔV et ΔC représentent la variation en valeur absolue des paramètres H, V et C et Co représente la pureté de la mèche par rapport à laquelle on désire évaluer la différence de couleur.

La puissance de la coloration (ΔE) est d'autant plus importante que le chiffre indiqué est élevé.

Les résultats sont donnés dans le tableau I ci-dessous :

**Tableau I**

| **EXEMPLE** | **Couleur des cheveux avant la teinture** | **Couleur des cheveux après la teinture** | **Puissance de la coloration** | | | |
|---|---|---|---|---|---|---|
| | | | ΔH | ΔV | ΔC | **ΔE** |
| **1** | 4,2 Y 5,6/1,6 | 8,6 PB 2,6/2,4 | 45,6 | 3,0 | 0,8 | **49,6** |
| **2(*)** | 4,2 Y 5,6/1,6 | 8,9 PB 2,9/1,6 | 45,3 | 2,7 | 0 | **45,2** |

| | | | | | | |
|---|---|---|---|---|---|---|
| (*) : Exemple ne faisant pas partie de l'invention. | | | | | | |

Ces résultats montrent que la composition tinctoriale prête à l'emploi de l'exemple 1 conforme à l'invention c'est à dire contenant, à titre de système oxydant, l'association de la choline oxydase et de la choline, conduit à une coloration plus puissante que celle obtenue avec la composition tinctoriale prête à l'emploi de l'exemple 2 ne faisant pas partie de l'invention car contenant, à titre de système oxydant, l'association de l'uricase et de l'acide urique. L'utilisation du système oxydant uricase / acide urique est notamment décrit dans la demande de brevet EP-A-0 310 675.

## Revendications

1. Composition prête à l'emploi, pour la teinture d'oxydation des fibres kératiniques et en particulier des fibres kératiniques humaines telles que les cheveux, **caractérisée par le fait qu'**elle comprend, dans un milieu approprié pour la teinture :
- au moins une base d'oxydation,
- de la choline oxydase,
- et au moins un donneur pour ladite choline oxydase.

2. Composition selon la revendication 1, **caractérisée par le fait que** la ou les bases d'oxydation sont choisies parmi les paraphénylènediamines, les bases doubles, les para-aminophénols, les ortho aminophénols et les bases d'oxydation hétérocycliques.

3. Composition selon la revendication 2, **caractérisée par le fait que** les paraphénylènediamines sont choisies parmi les composés de formule (I) suivante et leurs sels d'addition avec un acide : 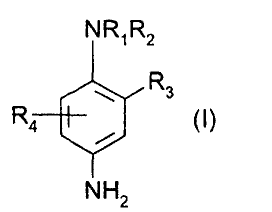 dans laquelle :
- R₁ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), alkyle en C₁-C₄ substitué par un groupement azoté, phényle ou 4'-aminophényle ;
- R₂ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄) ou alkyle en C₁-C₄ substitué par un groupement azoté ;
- R₃ représente un atome d'hydrogène, un atome d'halogène tel qu'un atome de chlore, de brome, d'iode ou de fluor, un radical alkyle en C₁-C₄, monohydroxyalkyle en C₁-C₄, hydroxyalcoxy en C₁-C₄, acétylaminoalcoxy en C₁-C₄, mésylaminoalcoxy en C₁-C₄ ou carbamoylaminoalcoxy en C₁-C₄,
- R₄ représente un atome d'hydrogène, d'halogène ou un radical alkyle en C₁-C₄.

4. Composition selon la revendication 3, **caractérisée par le fait que** les paraphénylènediamines de formule (I) sont choisies parmi la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphényiènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxy-éthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

5. Composition selon la revendication 2, **caractérisée par le fait que** la ou les bases doubles sont choisies parmi composés de formule (II) suivante, et leurs sels d'addition avec un acide : 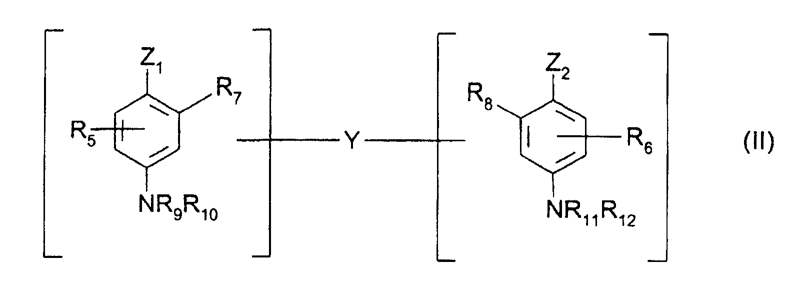 dans laquelle :
- Z₁ et Z₂, identiques ou différents, représentent un radical hydroxyle ou -NH₂ pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
- R₅ et R₆ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₇, R₈, R₉, R₁₀ R₁₁ et R₁₂, identiques ou différents, représentent un atome d'hydrogène, un bras de liaison Y ou un radical alkyle en C₁-C₄ ;
étant entendu que les composés de formule (II) ne comportent qu'un seul bras de liaison Y par molécule.

6. Composition selon la revendication 5, **caractérisée par le fait que** les composés de formule (II) sont choisis parmi le N,N'-bis-(β-hydroxyéthyl) N,N' bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

7. Composition selon la revendication 2, **caractérisée par le fait que** les paraaminophénols sont choisis parmi les composés de formule (III) suivante, et leurs sels d'addition avec un acide : 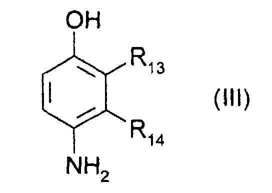 dans laquelle :
- R₁₃ représente un atome d'hydrogène ou d'halogène, un radical alkyle en C₁-C₄, monohydroxyalkyle en C₁-C₄, alcoxy(C₁-C₄)alkyle(C₁-C₄), aminoalkyle en C₁-C₄ ou hydroxyalkyl(C₁-C₄)aminoalkyle en C₁-C₄,
- R₁₄ représente un atome d'hydrogène ou d'halogène, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄),
étant entendu qu'au moins un des radicaux R₁₃ ou R₁₄ représente un atome d'hydrogène.

8. Composition selon la revendication 7, **caractérisée par le fait que** les paraaminophénols de formule (III) sont choisis parmi le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

9. Composition selon la revendication 2, **caractérisée par le fait que** les orthoaminophénols sont choisis parmi le 2-amino phénol, le 2-amino 5-méthyl phénol, le 2-amino 6-méthyl phénol, le 5-acétamido 2-amino phénol, et leurs sels d'addition avec un acide.

10. Composition selon la revendication 2, **caractérisée par le fait que** les bases d'oxydation hétérocycliques sont choisies parmi les dérivés pyridiniques, les dérivés pyrimidiniques, les dérivés pyrazoliques, les dérivés pyrazolo-pyrimidiniques, et leurs sels d'addition avec un acide.

11. Composition selon l'une quelconque des revendications précédentes, **caractérisée par le fait que** la ou les bases d'oxydation représentent de 0,0005 à 12 % en poids du poids total de la composition tinctoriale prête à l'emploi.

12. Composition selon la revendication 11, **caractérisée par le fait que** la ou les bases d'oxydation représentent de 0,005 à 6 % en poids du poids total de la composition tinctoriale prête à l'emploi.

13. Composition selon l'une quelconque des revendications précédentes, **caractérisée par le fait que** la choline oxydase représente de 0,01 à 20 % en poids du poids total de la composition tinctoriale prête à l'emploi.

14. Composition selon la revendication 13 **caractérisée par le fait que** la choline oxydase représente de 0,1 à 5 % en poids du poids total de la composition tinctoriale prête à l'emploi.

15. Composition selon l'une quelconque des revendications précédentes, **caractérisée par le fait que** le donneur pour ladite choline oxydase est choisi parmi la choline et ses sels d'addition avec un acide, et la bétaïne aldéhyde.

16. Composition selon l'une quelconque des revendications précédentes, **caractérisée par le fait que** le ou les donneurs représentent de 0,01 à 20 % en poids du poids total de la composition tinctoriale prête à l'emploi.

17. Composition selon la revendication 16, **caractérisée par le fait que** le ou les donneurs représentent de 0,1 à 5 % en poids du poids total de la composition tinctoriale prête à l'emploi.

18. Composition selon l'une quelconque des revendications précédentes, **caractérisée par le fait qu'**elle renferme un ou plusieurs coupleurs et/ou un ou plusieurs colorants directs.

19. Composition selon la revendication 18, **caractérisée par le fait que** les coupleurs sont choisis parmi les métaphénylènediamines, les métaaminophénols, les métadiphénols et les coupleurs hétérocycliques tels que par exemple les dérivés indoliques, les dérivés indoliniques, les dérivés de benzimidazole, les dérivés de benzomorpholine, les dérivés de sésamol, les dérivés pyridiniques, pyrimidiniques et pyrazoliques, et leurs sels d'addition avec un acide.

20. Composition selon l'une quelconque des revendications 18 ou 19, **caractérisée par le fait que** le ou les coupleurs représentent de 0,0001 à 10 % en poids du poids total de la composition tinctoriale prête à l'emploi.

21. Composition selon la revendication 20, **caractérisée par le fait que** le ou les coupleurs représentent de 0,005 à 5 % en poids du poids total de la composition tinctoriale prête à l'emploi.

22. Composition selon l'une quelconque des revendications précédentes, **caractérisée par le fait que** les sels d'addition avec un acide sont choisis parmi les chlorhydrates, les bromhydrates, les sulfates et les tartrates, les lactates et les acétates.

23. Composition selon l'une quelconque des revendications précédentes, **caractérisée par le fait que** le milieu approprié pour la teinture est constitué par de l'eau ou par un mélange d'eau et d'au moins un solvant organique.

24. Composition selon l'une quelconque des revendications précédentes, **caractérisée par le fait qu'**elle présente un pH compris 5 et 11.

25. Procédé de teinture des fibres kératiniques et en particulier des fibres kératiniques humaines telles que les cheveux, **caractérisé par le fait qu'**on applique sur lesdites fibres au moins une composition tinctoriale prête à l'emploi telle que définie dans l'une quelconque des revendications précédentes, pendant un temps suffisant pour développer la coloration désirée.

26. Procédé selon la revendication 25, **caractérisé par le fait qu'**il comporte une étape préliminaire consistant à stocker sous forme séparée, d'une part, une composition (A) comprenant, dans un milieu approprié pour la teinture, au moins une base d'oxydation, d'autre part, une composition (B) renfermant, dans un milieu approprié pour la teinture, de la choline oxydase en présence d'au moins un donneur pour ladite choline oxydase, puis à procéder à leur mélange au moment de l'emploi avant d'appliquer ce mélange sur les fibres kératiniques.

27. Dispositif à plusieurs compartiments ou "kit" de teinture, **caractérisé par le fait qu'**il comporte un premier compartiment renfermant la composition (A) telle que définie dans la revendication 26 et un second compartiment renfermant la composition (B) telle que définie dans la revendication 26.

## Patentansprüche

1. Gebrauchsfertige Zusammensetzung zum oxidativen Färben von Keratinfasern und insbesondere menschlichen Keratinfasern, wie dem Haar, **dadurch gekennzeichnet, dass** sie in einem zum Färben geeigneten Träger enthält:
- mindestens eine Oxidationsbase,
- die Cholin-Oxidase, und
- mindestens einen Donor für die Cholin-Oxidase.

2. Zusammensetzung nach Anspruch 1, **dadurch gekennzeichnet, dass** die Oxidationsbase(n) unter den p-Phenylendiaminen, Doppelbasen, p-Aminophenolen, o-Aminophenolen und den heterocyclischen Oxidationsbasen ausgewählt sind.

3. Zusammensetzung nach Anspruch 2, **dadurch gekennzeichnet, dass** die p-Phenylendiamine unter den Verbindungen der folgenden Formel (I) und deren Additionssalzen mit einer Säure ausgewählt sind: 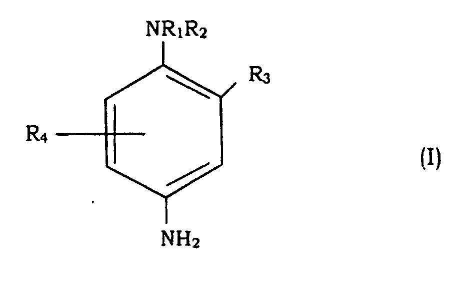 worin bedeuten:
- R₁ Wasserstoff, C₁₋₄-Alkyl, C₁₋₄-Monohydroxyalkyl, C₂₋₄-Polyhydroxyalkyl, C₁₋₄-Alkoxy-C₁₋₄-alkyl, eine mit einer stickstoffhaltigen Gruppe substituierte C₁₋₄-Alkylgruppe, Phenyl oder 4'-Aminophenyl,
- R₂ Wasserstoff, C₁₋₄-Alkyl, C₁₋₄-Monohydroxyalkyl, C₂₋₄-Polyhydroxyalkyl, C₁₋₄-Alkoxy-C₁₋₄-alkyl oder eine mit einer stickstoffhaltigen Gruppe substituierte C₁₋₄-Alkylgruppe,
- R₃ Wasserstoff, Halogen, wie Chlor, Brom, Iod oder Fluor, C₁₋₄-Alkyl, C₁₋₄-Monohydroxyalkyl, C₁₋₄-Hydroxyalkoxy, C₁₋₄-Acetylaminoalkoxy, C₁₋₄-Mesylaminoalkoxy oder C₁₋₄-Carbamoylaminoalkoxy, und
- R₄ Wasserstoff, Halogen oder C₁₋₄-Alkyl.

4. Zusammensetzung nach Anspruch 3, **dadurch gekennzeichnet, dass** die p-Phenylendiamine der Formel (I) unter p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-pphenylendiamin, 2,5-Dimethyl-p-phenylendiamin, N,N-Dimethyl-pphenylendiamin, N,N-Diethyl-p-phenylendiamin, N,N-Dipropyl-pphenylendiamin, 4-Amino-N,N-diethyl-3-methyl-anilin, N,N-Bis(β-hydroxyethyl)-p-phenylendiamin, 4-N,N-bis(β-Hydroxyethyl)amino-2-methyl-anilin, 4-N,N-bis(β-Hydroxyethyl)amino-2-chlor-anilin, 2-β-Hydroxyethyl-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl, β-hydroxyethyl)-pphenylendiamin, N-(β,γ-Dihydroxypropyl)-p-phenylendiamin, N-(4'-Aminophenyl)-p-phenylendiamin, N-Phenyl-p-phenylendiamin, 2-β-Hydroxyethyloxy-p-phenylendiamin, 2-β-Acetylaminoethyloxy-pphenylendiamin, N-(β-Methoxyethyl)-p-phenylendiamin und deren Additionssalzen mit einer Säure ausgewählt sind.

5. Zusammensetzung nach Anspruch 2, **dadurch gekennzeichnet, dass** die Doppelbasen unter den Verbindungen der folgenden Formel (II) und deren Additionssalze mit einer Säure ausgewählt sind: 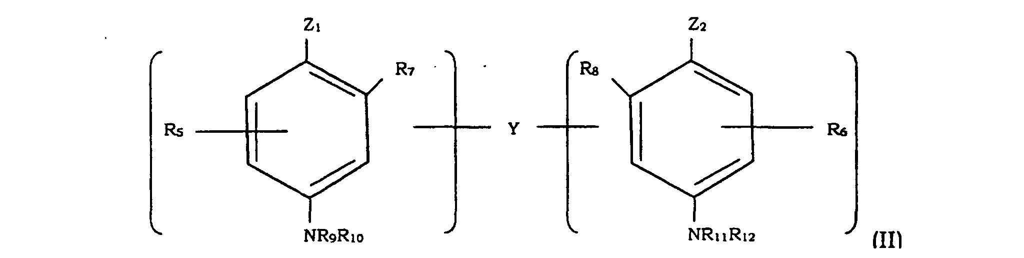 worin bedeuten:
- Z₁ und Z₂, die identisch oder voneinander verschieden sind, Hydroxy oder eine NH₂-Gruppe, die mit C₁₋₄-Alkyl oder einer Verbindungsgruppe Y substituiert sein kann,
- die Verbindungsgruppe Y eine geradkettige oder verzweigte Alkylengruppe mit 1 bis 14 Kohlenstoffatomen, die durch eine oder mehrere stickstoffhaltige Gruppen und/oder ein oder mehrere Heteroatome, wie Sauerstoff, Schwefel oder Stickstoff, unterbrochen oder abgeschlossen werden kann und gegebenenfalls mit einer oder mehreren Hydroxy- oder C₁₋₆-Alkoxygruppen substituiert sein kann,
- R₅ und R₆ Wasserstoff, Halogen, C₁₋₄-Alkyl, C₁₋₄-Monohydroxyalkyl, C₂₋₄-Polyhydroxyalkyl, C₁₋₄-Aminoalkyl oder eine Verbindungsgruppe Y,
- R₇, R₈, R₉, R₁₀, R₁₁ und R₁₂, die identisch oder voneinander verschieden sind, Wasserstoff, eine Verbindungsgruppe Y oder C₁₋₄-Alkyl, mit der Maßgabe, dass die Verbindungen der Formel (II) nur eine Verbindungsgruppe Y pro Molekül aufweisen.

6. Zusammensetzung nach Anspruch 5, **dadurch gekennzeichnet, dass** die Doppelbasen der Formel (II) unter N,N'-Bis(β-hydroxyethyl)-N,N'-bis-(4'-aminophenyl)-1,3-diaminopropanol, N,N'-Bis(β-hydroxyethyl)-N,N'-bis(4'-aminophenyl)-ethylendiamin, N,N'-Bis(4-aminophenyl)-tetramethylendiamin, N,N'-Bis(β-hydroxyethyl)-N,N'bis(4-aminophenyl)-tetramethylendiamin, N,N'-Bis(4-methylaminophenyl)-tetramethylendiamin, N,N'-Bis(ethyl)-N,N'-bis-(4'-amino, 3'methylphenyl)-ethylendiamin, 1,8-Bis(2,5-diamino-phenoxy)-3,5-dioxaoctan und deren Additionssalzen mit einer Säure ausgewählt sind.

7. Zusammensetzung nach Anspruch 2, **dadurch gekennzeichnet, dass** die p-Aminophenole unter den Verbindungen der folgenden Formel (III) und deren Additionssalze mit einer Säure ausgewählt sind: 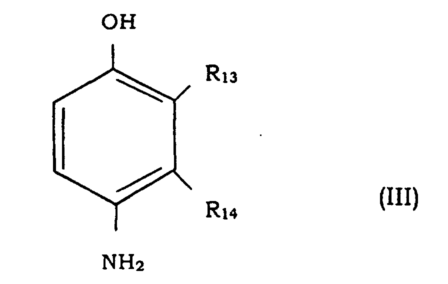 worin bedeuten:
- R₁₃ Wasserstoff, Halogen, C₁₋₄-Alkyl, C₁₋₄-Monohydroxyalkyl, C₁₋₄-Alkoxy-C₁₋₄-alkyl, C₁₋₄-Aminoalkyl oder C₁₋₄-Hydroxyalkyl-C₁₋₄aminoalkyl,
- R₁₄ Wasserstoff, Halogen, C₁₋₄-Alkyl, C₁₋₄-Monohydroxyalkyl, C₂₋₄-Polyhydroxyalkyl, C₁₋₄-Aminoalkyl, C₁₋₄-Cyanoalkyl oder C₁₋₄-Alkoxy-C₁₋₄-alkyl,
mit der Maßgabe, dass mindestens eine der Gruppen R₁₃ oder R₁₄ Wasserstoff bedeutet.

8. Zusammensetzung nach Anspruch 7, **dadurch gekennzeichnet, dass** die p-Aminophenole der Formel (III) unter p-Aminophenol, 4-Amino-3-methyl-phenol, 4-Amino-3-fluor-phenol, 4-Amino-3-hydroxymethyl-phenol, 4-Amino-2-methyl-phenol, 4-Amino-2-hydroxymethyl-phenol, 4-Amino-2-methoxymethyl-phenol, 4-Amino-2-aminomethyl-phenol, 4-Amino-2-(β-hydroxyethylaminomethyl)-phenol, 4-Amino-2-fluor-phenol und deren Additionssalzen mit einer Säure ausgewählt sind.

9. Zusammensetzung nach Anspruch 2, **dadurch gekennzeichnet, dass** die o-Aminophenole unter 2-Aminophenol, 2-Amino-5-methylphenol, 2-Amino-6-methyl-phenol, 5-Acetamido-2-amino-phenol und deren Additionssalzen mit einer Säure ausgewählt sind.

10. Zusammensetzung nach Anspruch 2, **dadurch gekennzeichnet, dass** die heterocyclischen Oxidationsbasen unter den Pyridinderivaten, Pyrimidinderivaten, Pyrazolderivaten, Pyrazolo-pyrimidinderivaten und deren Additionssalzen mit einer Säure ausgewählt sind.

11. Zusammensetzung nach einem der vorhergehenden Ansprüche, **dadurch gekennzeichnet, dass** die Oxidationsbase(n) 0,0005 bis 12 Gew.-% des Gesamtgewichts der gebrauchsfertigen Farbmittelzusammensetzung ausmachen.

12. Zusammensetzung nach Anspruch 11, **dadurch gekennzeichnet, dass** die Oxidationsbase(n) 0,005 bis 6 Gew.-% des Gesamtgewichts der gebrauchsfertigen Farbmittelzusammensetzung ausmachen.

13. Zusammensetzung nach einem der vorhergehenden Ansprüche, **dadurch gekennzeichnet, dass** die Cholin-Oxidase 0,01 bis 20 Gew.-% des Gesamtgewichts der gebrauchsfertigen Farbmittelzusammensetzung ausmacht.

14. Zusammensetzung nach Anspruch 13, **dadurch gekennzeichnet, dass** die Cholin-Oxidase 0,1 bis 5 Gew.-% des Gesamtgewichts der gebrauchsfertigen Farbmittelzusammensetzung ausmacht.

15. Zusammensetzung nach einem der vorhergehenden Ansprüche, **dadurch gekennzeichnet, dass** der Donor der Cholin-Oxidase unter Cholin und seinen Additionssalzen mit einer Säure und dem Betainaldehyd ausgewählt ist.

16. Zusammensetzung nach einem der vorhergehenden Ansprüche, **dadurch gekennzeichnet, dass** der Donor oder die Donoren 0,01 bis 20 Gew.-% des Gesamtgewichts der gebrauchsfertigen Farbmittelzusammensetzung ausmachen.

17. Zusammensetzung nach Anspruch 16, **dadurch gekennzeichnet, dass** der Donor oder die Donoren 0,1 bis 5 Gew.-% des Gesamtgewichts der gebrauchsfertigen Farbmittelzusammensetzung ausmachen.

18. Zusammensetzung nach einem der vorhergehenden Ansprüche, **dadurch gekennzeichnet, dass** sie einen oder mehrere Kuppler und/oder einen oder mehrere Direktfarbstoff enthält.

19. Zusammensetzung nach Anspruch 18, **dadurch gekennzeichnet, dass** die Kuppler unter den m-Phenylendiaminen, m-Aminophenolen, m-Dihydroxybenzolen und den heterocyclischen Kupplern, wie beispielsweise Indolderivaten, Indolinderivaten, Benzimidazolderivaten, Benzomorpholinderivaten, Sesamolderivaten, Pyridinderivaten, Pyrimidinderivaten und Pyrazolderivaten, und deren Additionssalzen mit einer Säure ausgewählt sind.

20. Zusammensetzung nach einem der Ansprüche 18 oder 19, **dadurch gekennzeichnet, dass** der oder die Kuppler 0,0001 bis 10 Gew.-% des Gesamtgewichts der gebrauchsfertigen Farbmittelzusammensetzung ausmachen.

21. Zusammensetzung nach Anspruch 20, **dadurch gekennzeichnet, dass** der oder die Kuppler 0,005 bis 5 Gew.-% des Gesamtgewichts der gebrauchsfertigen Farbmittelzusammensetzung ausmachen.

22. Zusammensetzung nach einem der vorhergehenden Ansprüche, **dadurch gekennzeichnet, dass** die Additionssalze mit einer Säure unter den Hydrochloriden, Hydrobromiden, Sulfaten, Tartraten, Lactaten und Acetaten ausgewählt sind.

23. Zusammensetzung nach einem der vorhergehenden Ansprüche, **dadurch gekennzeichnet, dass** das zum Färben geeignete Medium aus Wasser oder einem Gemisch von Wasser und mindestens einem organischen Lösungsmittel besteht.

24. Zusammensetzung nach einem der vorhergehenden Ansprüche, **dadurch gekennzeichnet, dass** sie einen pH-Wert im Bereich von 5 bis 11 aufweist.

25. Verfahren zum Färben von Keratinfasern und insbesondere menschlichen Keratinfasern, wie dem Haar, **dadurch gekennzeichnet, dass** auf die Fasern während einer Zeitspanne, die zur Entwicklung der gewünschten Färbung ausreichend ist, mindestens eine gebrauchsfertige Farbmittelzusammensetzung nach einem der vorhergehenden Ansprüche aufgebracht wird.

26. Verfahren nach Anspruch 25, **dadurch gekennzeichnet, dass** es einen vorbereitenden Schritt umfasst, der darin besteht, einerseits eine Zusammensetzung (A), die in einem zum Färben geeigneten Medium mindestens eine Oxidationsbase enthält, und andererseits eine Zusammensetzung (B) getrennt voneinander aufzubewahren, die in einem zum Färben geeigneten Medium die Cholin-Oxidase in Gegenwart mindestens eines Donors für die Cholin-Oxidase enthält, und diese bei der Anwendung zu vermischen, bevor das Gemisch auf die Fasern aufgebracht wird.

27. Vorrichtung mit mehreren Abteilungen oder "Kit" zum Färben, **dadurch gekennzeichnet, dass** sie eine erste Abteilung mit der in Anspruch 26 definierten Zusammensetzung (A) und eine zweite Abteilung mit der in Anspruch 26 definierten Zusammensetzung (B) enthält.

## Claims

1. Ready-to-use composition for the oxidation dyeing of keratin fibres, and in particular of human keratin fibres such as the hair, **characterized in that** it comprises, in a medium which is suitable for dyeing:
- at least one oxidation base,
- choline oxidase,
- and at least one donor for the said choline oxidase.

2. Composition according to Claim 1, **characterized in that** the oxidation base(s) is (are) chosen from para-phenylenediamines, double bases, para-aminophenols, ortho-aminophenols and heterocyclic oxidation bases.

3. Composition according to Claim 2, **characterized in that** the para-phenylenediamines are chosen from the compounds of formula (I) below, and the addition salts thereof with an acid: 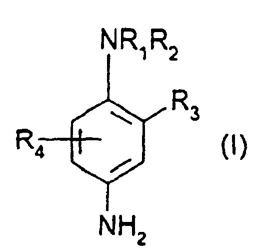 in which:
- R₁ represents a hydrogen atom, a C₁-C₄ alkyl radical, a C₁-C₄ monohydroxyalkyl radical, a C₂-C₄ polyhydroxyalkyl radical, a (C₁-C₄)alkoxy(C₁-C₄)alkyl radical, a C₁-C₄ alkyl radical substituted with a nitrogenous group, a phenyl radical or a 4-aminophenyl radical;
- R₂ represents a hydrogen atom, a C₁-C₄ alkyl radical, a C₁-C₄ monohydroxyalkyl radical, a C₂-C₄ polyhydroxyalkyl radical, a (C₁-C₄)alkoxy(C₁-C₄)alkyl radical or a C₁-C₄ alkyl radical substituted with a nitrogenous group;
- R₃ represents a hydrogen atom, a halogen atom such as a chlorine, bromine, iodine or fluorine atom, a C₁-C₄ alkyl radical, a C₁-C₄ monohydroxyalkyl radical, a C₁-C₄ hydroxyalkoxy radical, an acetylamino(C₁-C₄)alkoxy radical, a C₁-C₄ mesylaminoalkoxy radical or a carbamoylamino(C₁-C₄)alkoxy radical,
- R₄ represents a hydrogen or halogen atom or a C₁-C₄ alkyl radical.

4. Composition according to Claim 3, **characterized in that** the para-phenylenediamines of formula (I) are chosen from para-phenylenediamine, paratolylenediamine, 2-chloro-para-phenylenediamine, 2,3-dimethyl-para-phenylenediamine, 2,6-dimethyl-para-phenylenediamine, 2,6-diethyl-para-phenylenediamine, 2,5-dimethyl-para-phenylenediamine, N,N-dimethyl-para-phenylenediamine, N,N-diethyl-para-phenylenediamine, N,N-dipropyl-para-phenylenediamine, 4-amino-N,N-diethyl-3-methylaniline, N,N-bis(β-hydroxyethyl)-para-phenylenediamine, 4-N,N-bis(β-hydroxyethyl)amino-2-methylaniline, 4-N,N-bis(β-hydroxyethyl)amino-2-chloroaniline, 2-β-hydroxyethyl-para-phenylenediamine, 2-fluoro-para-phenylenediamine, 2-isopropyl-para-phenylenediamine, N-(β-hydroxypropyl)-para-phenylenediamine, 2-hydroxymethyl-para-phenylenediamine, N,N-dimethyl-3-methyl-para-phenylenediamine, N,N-(ethyl-β-hydroxyethyl)-para-phenylenediamine, N-(β,γ-dihydroxypropyl)-para-phenylenediamine, N-(4'-aminophenyl)-para-phenylenediamine, N-phenyl-para-phenylenediamine, 2-β-hydroxyethyloxy-para-phenylenediamine, 2-β-acetylaminoethyloxy-para-phenylenediamine and N-(β-methoxyethyl)-para-phenylenediamine, and the addition salts thereof with an acid.

5. Composition according to Claim 2, **characterized in that** the double base(s) is (are) chosen from compounds of formula (II) below, and the addition salts thereof with an acid: 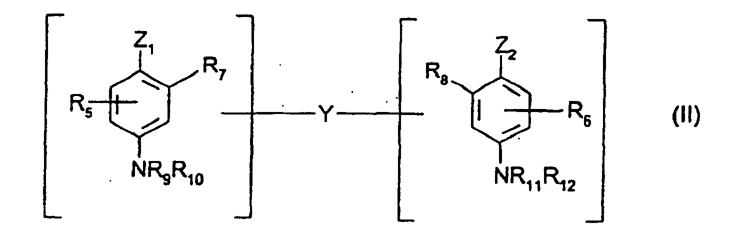 in which:
- Z₁ and Z₂, which may be identical or different, represent a hydroxyl or -NH₂ radical which may be substituted with a C₁-C₄ alkyl radical or with a linker arm Y;
- the linker arm Y represents a linear or branched alkylene chain containing from 1 to 14 carbon atoms, which may be interrupted by or terminated with one or more nitrogenous groups and/or by one or more hetero atoms such as oxygen, sulphur or nitrogen atoms, and optionally substituted with one or more hydroxyl or C₁-C₆ alkoxy radicals;
- R₅ and R₆ represent a hydrogen or halogen atom, a C₁-C₄ alkyl radical, a C₁-C₄ monohydroxyalkyl radical, a C₂-C₄ polyhydroxyalkyl radical, a C₁-C₄ aminoalkyl radical or a linker arm Y;
- R₇, R₈, R₉, R₁₀, R₁₁ and R₁₂, which may be identical or different, represent a hydrogen atom, a linker arm Y or a C₁-C₄ alkyl radical;
it being understood that the compounds of formula (II) contain only one linker arm Y per molecule.

6. Composition according to Claim 5, **characterized in that** the compounds of formula (II) are chosen from N,N'-bis(β-hydroxyethyl)-N,N'-bis(4'-aminophenyl)-1,3-diaminopropanol, N,N'-bis(β-hydroxyethyl)-N,N'-bis(4'-aminophenyl)ethylenediamine, N,N'-bis(4-aminophenyl)tetramethylenediamine, N,N'-bis(β-hydroxyethyl)-N,N'-bis(4-aminophenyl)-tetramethylenediamine, N,N'-bis(4-methylaminophenyl)tetramethylenediamine, N,N'-bis(ethyl)-N,N'-bis(4'-amino-3'-methylphenyl)-ethylenediamine and 1,8-bis(2,5-diaminophenoxy)-3,5-dioxaoctane, and the addition salts thereof with an acid.

7. Composition according to Claim 2, **characterized in that** the para-aminophenols are chosen from the compounds of formula (III) below, and the addition salts thereof with an acid: 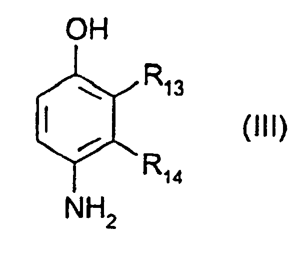 in which:
- R₁₃ represents a hydrogen or halogen atom or a C₁-C₄ alkyl, C₁-C₄ monohydroxyalkyl, (C₁-C₄)-alkoxy(C₁-C₄)alkyl, C₁-C₄ aminoalkyl or hydroxy(C₁-C₄)alkylamino(C₁-C₄)alkyl radical,
- R₁₄ represents a hydrogen or halogen atom or a C₁-C₄ alkyl, C₁-C₄ monohydroxyalkyl, C₂-C₄ polyhydroxyalkyl, C₁-C₄ aminoalkyl, C₁-C₄ cyanoalkyl or (C₁-C₄)alkoxy(C₁-C₄)alkyl radical, it being understood that at least one of the radicals R₁₃ or R₁₄ represents a hydrogen atom.

8. Composition according to Claim 7, **characterized in that** the para-aminophenols of formula (III) are chosen from para-aminophenol, 4-amino-3-methylphenol, 4-amino-3-fluorophenol, 4-amino-3-hydroxymethylphenol, 4-amino-2-methylphenol, 4-amino-2-hydroxymethylphenol, 4-amino-2-methoxymethylphenol, 4-amino-2-aminomethylphenol, 4-amino-2-(β-hydroxyethylaminomethyl)phenol and 4-amino-2-fluorophenol, and the addition salts thereof with an acid.

9. Composition according to Claim 2, **characterized in that** the ortho-aminophenols are chosen from 2-aminophenol, 2-amino-5-methylphenol, 2-amino-6-methylphenol and 5-acetamido-2-aminophenol, and the addition salts thereof with an acid.

10. Composition according to Claim 2, **characterized in that** the heterocyclic oxidation bases are chosen from pyridine derivatives, pyrimidine derivatives, pyrazole derivatives and pyrazolopyrimidine derivatives, and the addition salts thereof with an acid.

11. Composition according to any one of the preceding claims, **characterized in that** the oxidation base(s) represent(s) from 0.0005 to 12% by weight relative to the total weight of the ready-to-use dye composition.

12. Composition according to Claim 11, **characterized in that** the oxidation base(s) represent(s) from 0.005 to 6% by weight relative to the total weight of the ready-to-use dye composition.

13. Composition according to any one of the preceding claims, **characterized in that** the choline oxidase represents from 0.01 to 20% by weight relative to the total weight of the ready-to-use dye composition.

14. Composition according to Claim 13, **characterized in that** the choline oxidase represents from 0.1 to 5% by weight relative to the total weight of the ready-to-use dye composition.

15. Composition according to any one of the preceding claims, **characterized in that** the donor for the said choline oxidase is chosen from choline and its addition salts with an acid, and betaine aldehyde.

16. Composition according to any one of the preceding claims, **characterized in that** the donor(s) represent(s) from 0.01 to 20% by weight relative to the total weight of the ready-to-use dye composition.

17. Composition according to Claim 16, **characterized in that** the donor(s) represent(s) from 0.1 to 5% by weight relative to the total weight of the ready-to-use dye composition.

18. Composition according to any one of the preceding claims, **characterized in that** it contains one or more couplers and/or one or more direct dyes.

19. Composition according to Claim 18, **characterized in that** the couplers are chosen from metaphenylenediamines, meta-aminophenols, metadiphenols and heterocyclic couplers such as, for example, indole derivatives, indoline derivatives, benzimidazole derivatives, benzomorpholine derivatives, sesamol derivatives and pyridine, pyrimidine and pyrazole derivatives, and the addition salts thereof with an acid.

20. Composition according to either of Claims 18 and 19, **characterized in that** the coupler(s) represent(s) from 0.0001 to 10% by weight relative to the total weight of the ready-to-use dye composition.

21. Composition according to Claim 20, **characterized in that** the coupler(s) represent(s) from 0.005 to 5% by weight relative to the total weight of the ready-to-use dye composition.

22. Composition according to any one of the preceding claims, **characterized in that** the addition salts with an acid are chosen from the hydrochlorides, hydrobromides, sulphates, tartrates, lactates and acetates.

23. Composition according to any one of the preceding claims, **characterized in that** the medium which is suitable for dyeing consists of water or of a mixture of water and at least one organic solvent.

24. Composition according to any one of the preceding claims, **characterized in that** it has a pH of between 5 and 11.

25. Process for dyeing keratin fibres, and in particular human keratin fibres such as the hair, **characterized in that** at least one ready-to-use dye composition as defined in any one of the preceding claims is applied to the said fibres for a period which is sufficient to develop the desired coloration.

26. Process according to Claim 25, **characterized in that** it includes a first step which consists in separately storing, on the one hand, a composition (A) comprising, in a medium which is suitable for dyeing, at least one oxidation base and, on the other hand, a composition (B) containing, in a medium which is suitable for dyeing, choline oxidase in the presence of at least one donor for the said choline oxidase, and then in mixing them together at the time of use, before applying this mixture to the keratin fibres.

27. Multi-compartment dyeing device or "kit", **characterized in that** it includes a first compartment containing composition (A) as defined in Claim 26 and a second compartment containing composition (B) as defined in Claim 26.
